**Europäisches Patentamt**

**European Patent Office**

**Office européen des brevets**

(19)

(11) Veröffentlichungsnummer : **0 055 383**
**B1**

(12) **EUROPÄISCHE PATENTSCHRIFT**

(45) Veröffentlichungstag der Patentschrift :
**03.04.85**

(21) Anmeldenummer : **81109287.3**

(22) Anmeldetag : **29.10.81**

(51) Int. Cl.⁴ : **A 61 K   6/04**

(54) **Aufsinterbare Grundmasse zur Herstellung einer Zwischenschicht zwischen einer hochschmelzenden Dentalmetall-Legierung und Zahnporzellan.**

(30) Priorität : **09.12.80 DE 3046334**

(43) Veröffentlichungstag der Anmeldung :
**07.07.82 Patentblatt 82/27**

(45) Bekanntmachung des Hinweises auf die Patenterteilung : **03.04.85 Patentblatt 85/14**

(84) Benannte Vertragsstaaten :
**AT BE CH DE FR GB IT LI NL SE**

(56) Entgegenhaltungen :
**DE-A- 2 525 274**
**FR-A-   996 609**
**US-A- 4 181 757**

(73) Patentinhaber : **Etablissement Dentaire IVOCLAR**
**FL-9494 Schaan (LI)**

(72) Erfinder : **Shoher, Itzhak, Dr.**
**50, Shlomo Hamelech St.**
**Tel Aviv (IL)**
Erfinder : **Whiteman, Aaron**
**50, Shlomo Hamelech St.**
**Tel Aviv (IL)**

(74) Vertreter : **Reitzner, Bruno, Dr. et al**
**Patentanwälte Dipl.-Ing. R. Splanemann Dr. B.**
**Reitzner Tal 13**
**D-8000 München 2 (DE)**

**Beschreibung**

Die Erfindung betrifft eine aufsinterbare Grundmasse zur Herstellung einer Zwischenschicht zwischen einer hochschmelzenden Dentalmetall-Legierung, insbesonder einer Nichtedelmetall-Legierung, und Zahnporzellan sowie ein Verfahren zur Herstellung einer solchen Zwischenschicht.

Aufsinterbare Grundmassen werden in der Dentaltechnik dazu verwendet, um die Haftung zwischen einem metallischen Überzug, gewöhnlich aus einer Nichtedelmetall-Legierung, und einem nachträglich aufgebrannten Zahnporzellan zu verbessern. Bei der Herstellung von Zahnrestaurationen, insbesondere von Kronen und Brücken, wird auf einen metallischen Unterbau, z. B. auf eine Krone, die speziell vorbehandelt und gereinigt werden muß, Zahnporzellan aufgetragen und schichtweise bei hohen Temperaturen eingebrannt, so daß ein ästhetisch einwandfreier Zahnersatz entsteht.

Es hat sich aber gezeigt, daß die Haftung des Zahnporzellans am Metall in vielen Fällen unbefriedigend ist und in hohem Maße vom Können des Zahntechnikers abhängt. Dies ist besonders dann der Fall, wenn als Nichtedelmetall-Legierung die im Handel befindlichen Legierungen auf der Basis von Nickel, Chrom und/oder Kobalt verwendet werden, in denen noch andere Zusätze wie Molybdän, Aluminium, Silicium, Mangan, Eisen usw. enthalten sein können. Unedle Legierungen werden heute aus Kostengründen vermehrt verwendet und sind in der Literatur hinreichend beschrieben.

Um die Haftung zwischen Metall und Zahnporzellan zu verbessern, wurden bereits verschiedene Bindemittel vorgeschlagen. Nach der DE-OS 25 25 274 wurden Goldpulver, Zahnporzellan, Zirkondioxid und flüssiges Flußmittel in bestimmten Zusammensetzungen als Bindemittel zwischen Zahnporzellan und Metall verwendet. Das flüssige Flußmittel enthält dabei noch Trägerstoffe, wie Glycerin oder Alkohol. Es kann aus Boroxid oder den Salzen davon, wie Natriumborat, oder den Oxiden der leichten Elemente, wie Lithiumoxid, Natriumoxid usw. bestehen. Während des Brennens bildet das Flußmittel zusammen mit dem Zahnporzellan und den Oxiden auf der Legierung eine zähe Zwischenschicht aus Oxiden geringerer Löslichkeit, an der das Zahnporzellan während des nachfolgenden Brennens gut haftet.

Aus der DE-OS 26 12 554 ist als Haftmittelzusammensetzung eine Mischung aus Bor und Aluminium zusammen mit einem organischen Trägerbestandteil bekannt. Auf einen gereinigten Metallunterbau wird dabei eine gleichmäßige Schicht aus z. B. in Vaseline dispergiertem Bor und Aluminium aufgebracht, wonach das beschichtete Metallteil schnell auf eine hohe Temperatur gebracht und das Bindemittel eingebrannt wird. Die beschichtete Oberfläche wird nach dem Abkühlen gereinigt, und das Zahnporzellan kann in üblicher Weise aufgebrannt werden.

Ferner ist aus der DE-OS 26 26 092 ein Bindemittel bekannt, das ein Aluminiumpulver, ein Glaspulver und ein organisches Trägermittel enthält. Die Trägerkomponente erzeugt während des Sinterns eine reduzierende Atmosphäre. Das Glas ist ein Gemisch von nicht kristallinen Oxiden, zu denen $SiO_2$, $Al_2O_3$, $Na_2O$, $CaO$, $MgO$, $K_2O$, $SnO_2$, $Li_2O$, $B_2O_3$, $Fe_2O_3$, $BaO$ gehören.

Zum Stand der Technik (DE-OS 26 32 871) gehören ferner Verfahren, bei denen auf das Metallsubstrat ein Gemisch aus Ni-Cr-Al und Keramik (Al$_2$O$_3$, ZrO$_2$, TiO$_2$) mit einem Plasma-Brenner aufgedampft wird.

Auch die Erzeugung einer Zwischenschicht, die aus Au, Porzellan und $ZrO_2$ und einem Flußmittel besteht, ist aus der US-PS 41 81 757 bekannt.

Alle vorstehend beschriebenen Haft- oder Bindemittel sind jedoch in ihrer Wirkungsweise noch nicht zufriedenstellend hinsichtlich der Haftverbesserung zwischen dem Metallunterbau und dem Zahnporzellan. Teils sind die Verfahren dazu aufwendig und teuer, teils verbessern die verwendeten Bindemittel nur die chemische Bindung zwischen Metall und Porzellan. Aufgabe der Erfindung ist es daher, eine Grundmasse bereitzustellen, die nach dem Aufsintern die Haftung zwischen einer hochschmelzenden Dentalmetall-Legierung, insbesonder einer Nichtedelmetall-Legierung, und Zahnporzellan dadurch verbessert, daß sie nicht nur durch chemische Bindungsmechanismen, sondern auch durch mechanische Retentionen wirkt.

Die Aufgabe wird erfindungsgemäß durch eine aufsinterbare Grundmasse der eingangs definierten Gattung gelöst, die durch folgende Komponenten gekennzeichnet ist :

(a) mindestens ein hochschmelzendes, unedles Dentalmetall bzw. eine hochschmelzende Dentalmetall-Legierung ;

(b) Gold bzw. eine Goldverbindung, die sich zu metallischem Gold zersetzt, oder eine Goldlegierung ;

(c) mindestens ein Flußmittel ; und

(d) Aluminium und/oder Silber oder eine Aluminium-Silber-Legierung bzw. eine Silberverbindung, die sich zu metallischem Silber zersetzt.

Die erfindungsgemäße Grundmasse kann zusätzlich

(e) Kieselsäure und/oder

(f) Zirkondioxid enthalten.

Unter « hochschmelzenden » unedlen Deutab-metallen bzw.-metall-Legierungen versteht man Metalle und Legierungen, die bei den Temperaturen, bei denen Zahnporzellane bzw. Glasuren üblicherweise aufgesintert werden, thermisch beständig sind. Im allgemeinen liegen die Schmelzpunkte dieser hochschmelzenden Metalle bzw. Metall-Legierungen oberhalb etwa 800 °C, insbesondere oberhalb 1 000 °C. In diesem Temperaturbereich liegen üblicherweise auch die Brenntemperaturen von Zahnporzellan. Die hochschmelzenden Dentalmetalle bzw. Dentalmetall-Legierungen werden in den unterschiedlichsten Zusammensetzungen als Handelsprodukte vertrieben. Beispiele für Nichtedelmetalle sind Nickel, Kobalt, Chrom, Molybdän, Wolfram, Mangan, Tantal, Titan und Zirkon sowie deren Legierungur.

Bei den erfindungsgemäß verwendbaren Gold- bzw. Silberverbindungen, die sich zu metallischem Gold bzw. Silber zersetzen, handelt es sich in erster Linie um die entsprechenden Halogenide. Diese zersetzen sich bei den üblichen Sintertemperaturen oder darunter. Die Goldhalogenide zersetzen sich bei noch tieferen Temperaturen. Eine weitere thermisch zersetzbare Silberverbindung ist zum Beispiel Silberoxid.

Wird die erfindungsgemäße Grundmasse auf eine hochschmelzende Dentalmetall-Legierung, z. B. auf einen Metallunterbau wie eine Krone oder Brücke, aufgesintert, so erhält man nach einer weiteren Behandlung eine Zwischenschicht, auf die Zahnporzellan aufgebrannt wird, wodurch man ein Zahnrestaurationsteil erhält, welches wesentlich größeren Belastungen ausgesetzt werden kann als bei Nichtverwendung der erfindungsgemäßen Grundmasse. Insbesondere wird bei Verwendung der erfindungsgemäßen Grundmasse die Gefahr des Abplatzens von Zahnporzellan, wie es bei hohen unvorhergesehenen Kaubelastungen immer wieder vorkommt, vermindert.

Das Verfahren zur Herstellung einer Zwischenschicht zwischen einer hochschmelzenden Dentalmetall-Legierung (Nichtedelmetall-Legierung), und Zahnporzellan ist ebenfalls Gegenstand der vorliegenden Erfindung. Dieses Verfahren ist dadurch gekennzeichnet, daß man eine erfindungsgemäße Grundmasse mit einer Trägerflüssigkeit (A), in der die Komponente (c) mindestens teilweise löslich ist, vermischt, die Mischung auf die hochschmelzende Dentalmetall-Legierung aufbringt, trocknet und aufsintert und die aufgesinterte Grundmasse mit einer Flüssigkeit (B), in der die nichtmetallischen Komponenten der Grundmasse mindestens teilweise löslich sind, behandelt.

Vorzugsweise liegen in der erfindungsgemäßen Grundmasse mindestens die Komponenten (a), (b) und (d) sowie gegebenenfalls die Komponenten (e) und (f) in Form von Teilchen vor. Auch die Komponente (c), d. h. das Flußmittel, kann in Teilchenform vorliegen, doch kann diese Komponente auch eine Matrix darstellen, in welcher die anderen Komponenten eingebettet sind. Ferner kann die Komponente (c) auch in einem Lösungsmittel gelöst sein. Dies gilt auch für die Gold- bzw. Silberverbindungen, die gegebenenfalls in den Komponenten (b) bzw. (d) verwendet werden können.

Die teilchenförmige Grundmasse ist vorzugsweise pulverförmig, wobei die Teilchen eine regelmäßige (z. B. eine kugelförmige) und/oder eine unregelmäßige Struktur (Späne, Plättchen) haben können. Die Teilchengröße der Komponenten liegt vorzugsweise zwischen etwa 10 und 200 μm, insbesondere zwischen etwa 20 und 60 μm. Beispielsweise haben die Komponenten folgende Teilchengrößen (in μm) :

(a) 20 bis 50 ;
(b) 30 bis 200, vorzugsweise weniger als 150 ;
(c) weniger als 150 ;
(d) 20 bis 200, vorzugsweise weniger als 50 ;
(e) 20 bis 150 ;
(f) weniger als 50.

Vorzugsweise hat die erfindungsgemäße Grundmasse etwa folgende Zusammensetzung (in Gew.-%) :

(a) 10 bis 70 ;
(b) 10 bis 65 ;
(c) 5 bis 50 ;
(d) 0,6 bis 20 ;
(e) 0 bis 20 ;
(f) 0 bis 12.

Eine besonders bevorzugte Zusammensetzung (in Gew.-%) ist wie folgt :

(a) 20 bis 50, insbesondere 35 ;
(b) 35 bis 50, insbesondere 42 ;
(c) 15 bis 30, insbesondere 21 ;
(d) 1,5 bis 2, insbesondere 1,8 ;
(e) 0 bis 20 ;
(f) 0 bis 8.

Vorzugsweise enthält die Komponente (a) in der Grundmasse Nickel, Kobalt oder Chrom bzw. Gemische davon. Derartige hochschmelzende Metalle bzw. Legierungen sind an sich bekannt und enthalten üblicherweise noch Zusätze von Mo, Al, Si, Mn und Fe, die für den jeweiligen Verwendungszweck vorteilhafte Eigenschaften ergeben. Eine Legierungszusammensetzung für die Komponente (a) (in Gew.-%) ist wie folgt :

Cr 10 bis 20 ;
Mo 1 bis 7 ;

3

Al 1 bis 5 ;
Si 0,5 bis 2 ;
Mn 2 bis 5 ;
Fe 0 bis 5 ;
Co 0,1 bis 1, Ni-Rest.

Innerhalb dieser Bereiche hat eine bevorzugte Komponente (a) folgende Zusammensetzung (in Gew.-%) :

Cr 17 ; Mo 5 ; Al 4 ; Si 1 ; Mn 3 ; Fe 0,5 ; Co 0,5 ; Ni 69.

Die bevorzugt verwendete Legierungskomponente (a) besteht aus unregelmäßig geformten Spänen mit einer Länge von etwa 50 bis 150 $\mu$m und einer Dicke von etwa 20 bis 60 $\mu$m. Die Komponente (a) wird innerhalb des vorstehend angegebenen Bereichs in einem besonders ausgewählten Bereich von etwa 20 und 40 Gew.-% verwendet.

Die Komponente (b) kann aus Gold bzw. einer Goldlegierung bestehen, wobei der Goldanteil der Legierung mindestens 50 Gew.-% betragen soll. Weitere Legierungsbestandteile können Ag, Cu, Pt und Pd sein. Üblicherweise hat die Komponente (b) folgende Zusammensetzung (in Gew.-%) :

Au 50 bis 100 ; Ag 0 bis 25 ; Cu 0 bis 15 ; Pt 0 bis 10 ; Pd 0 bis 10.

Eine bevorzugte Zusammensetzung (in Gew.-%) ist wie folgt :

Au 76 ; Ag 15 ; Cu 8 ; Pt 0,4 ; Pd 0,6.

Die Teilchengröße dieser Legierungen liegt üblicherweise zwischen etwa 30 und 200 $\mu$m. Vorzugsweise werden Späne verwendet.

Als Komponente (c) können die bekannten Flußmittel Verwendung finden. Es sind dies zum Beispiel Boroxid, die Alkaliborate, -carbonate, -fluoride, Fluoroborate und -chloride, insbesondere des Natriums und des Kaliums. Grundsätzlich werden diejenigen Flußmittel bevorzugt, die während des Sinterprozesses die Oxide an der Oberfläche des Metallunterbaus entfernen. Als bevorzugte Verbindungen seien Borax und Natriumfluoroborat genannt. Die Komponente (c) kann innerhalb der vorstehend genannten Bereiche speziell in einem Bereich von etwa 10 bis 20 Gew.-% eingesetzt werden.

Die Komponente (d) hat einen Schmelzpunkt von unter 1 000 °C, wobei der Schmelzpunkt vorzugsweise dadurch erniedrigt wird, daß Al-Ag-Legierungen eingesetzt werden.

Als wahlweise verwendete Komponente (e) wird vorzugsweise ein $SiO_2$-Pulver mit einer Teilchengröße von weniger als 50 $\mu$m verwendet. Es können aber auch anstelle der reinen $SiO_2$-Komponente Zahnporzellane verwendet werden, die aber $SiO_2$ in größeren Mengen enthalten sollen. Der $SiO_2$-Anteil in diesen Zahnporzellanen sollte mindestens 40 Gew.-% betragen.

Der Einsatz der Komponente (f) ist ebenfalls wahlweise, wobei die Teilchengröße weniger als 50 $\mu$m betragen sollte.

Eine bevorzugte Zusammensetzung der aufsinterbaren Grundmasse ist wie folgt :

a) 35 Gew.-% der vorstehend als Beispiel genannten Nichtedelmetall-Legierung ;
b) 42 Gew.-% der vorstehend als Beispiel genannten Goldlegierung ;
c) 21 Gew.-% Borax ;
d)  2 Gew.-% Al-Pulver.

Mindestens ein Teil der Komponenten der erfindungsgemäßen Grundmasse kann in einer Trägerflüssigkeit suspendiert sein und in dieser Form in den Handel gebracht werden. Die Grundmasse kann aber auch unmittelbar vor Gebrauch mit der Trägerflüssigkeit angemischt werden.

Die pulverförmige Grundmasse wird vorzugsweise in Form einer Suspension in der Trägerflüssigkeit (A) auf die Metallunterlage aufgetragen. Dies erfolgt im allgemeinen durch Pinseln, aber auch durch Tauchen oder Sprühen. Als Trägerflüssigkeit können anorganische oder vorzugsweise organische Flüssigkeiten verwendet werden, wobei die Trägerflüssigkeit das Flußmittel (c) mindestens teilweise löst. Bevorzugt verwendet man als Trägerflüssigkeit (A) ein Gemisch aus Glycerin und Äthanol, insbesondere im Gewichtsverhältnis von etwa 1 : 1.

Man kann die pulverförmige Grundmasse mit dem Gemisch aus Glycerin und Äthanol so vermischen, daß eine cremeartige Suspension entsteht. Diese kann beispielsweise in einer Schichtstärke von etwa 0,2 bis 0,5 mm auf die hochschmelzende Dentalmetall-Legierung, die z. B. die Form einer Krone hat, aufgebracht werden. Die so beschichtete Krone wird in einem Brennofen langsam getrocknet, bevor die Schicht vorzugsweise im Vakuum aufgesintert wird. Das Aufsintern erfolgt bei ansteigenden Temperaturen, wobei der Temperaturanstieg z. B. 60 bis 120 °C pro Minute, vorzugsweise etwa 120 °C pro Minute betragen kann. Die Endtemperatur beträgt etwa 800 bis 1 200 °C, vorzugsweise etwa 950 °C. Nach einer Sinterzeit von etwa 2 Minuten wird das Vakuum im Ofen gebrochen, der Ofen geöffnet, die Krone entnommen und bei Raumtemperatur abgekühlt.

4

Bevor man mit dem Aufbrennen des Zahnporzellans beginnt, wird die aufgesinterte Zwischenschicht mit einer Flüssigkeit (B), in der die nichtmetallischen Komponenten der Grundmasse, insbesondere das Flußmittel, mindestens teilweise löslich sind, behandelt.

Als Behandlungsflüssigkeit (B) verwendet man vorzugsweise eine verdünnte Mineralsäure oder ein Gemisch aus verdünnten Mineralsäuren. Die verdünnte Mineralsäure kann etwa 5 bis 10 % HCl und/oder 5 bis 10 % HF in wäßriger Lösung enthalten. Um die Oberfläche der aufgebrachten Schicht besonders sorgfältig zu reinigen, kann man die Behandlung mit der Flüssigkeit (B) in einem Ultraschallbad durchführen. Durch die Säure werden die nichtmetallischen Bestandteile an der Oberfläche entfernt, d. h. insbesondere das Flußmittel (c) und die durch das Flußmittel gelösten Metalloxide. Man erhält eine sehr rauhe Oberfläche, die mit einer sehr dünnen Goldschicht überzogen ist.

Man kann sich den Vorgang ungefähr so erklären, daß das Flußmittel (zusammen mit der gegebenenfalls verwendeten Kieselsäure) die an der Metalloberfläche befindlichen Oxide während des Sinterungsprozesses löst, so daß die Komponente (d) an die Komponente (a) bzw. an die hochschmelzende Dentalmetall-Legierung des Unterbaus gelangen kann, so daß zunächst eine dünne Haftvermittlerschicht aus Aluminium bzw. Silber entsteht, auf der sich eine dünne Goldschicht ausbreitet. Die Ausbreitung dieser Goldschicht wird zu einem gewissen Grad durch das gegebenenfalls mitverwendete $ZrO_2$ erleichtert. Die Teilchen der Komponente (a) sind also mit Hilfe der dünnen Goldschicht aus der Komponente (b) mit der Dentalmetall-Legierung des Unterbaus verbunden. Durch die Oberflächenrauhigkeit des Sinterkörpers wird die erforderliche mechanische Retention für das Zahnporzellan erzielt. Durch die Behandlung mit der Flüssigkeit (B) werden nicht nur die nichtmetallischen Bestandteile, wie das Flußmittel, entfernt, sondern auch die im Flußmittel eingebetteten Metallteilchen, die am Sinterprozeß nur unvollständig beteiligt waren.

Als hochschmelzende Dentalmetall-Legierung für den Unterbau verwendet man vorzugsweise eine Legierung mit Nickel, Kobalt und/oder Chrom, bzw. von Gemischen davon als Hauptbestandteilen, deren thermischer Ausdehnungskoeffizient dem des Zahnporzellans angepaßt ist. Im allgemeinen hat diese Legierung etwa die gleiche Zusammensetzung wie die Komponente (a) der erfindungsgemäßen Grundmasse.

Eine bevorzugt verwendete hochschmelzende Dentalmetall-Legierung für den Unterbau (bzw. für die Komponente (a)) hat etwa folgende Zusammensetzung (in Gew.-%) :

Ni und/oder Co 60 bis 85 ; Cr 11 bis 32 ; Fe 0,2 bis 0,6 ; Al 0 bis 5 ; Mo 1,5 bis 6 ; Si 0 bis 1,2 ; Be 0 bis 1,6 ; Cu 0 bis 0,2 ; Mg 0 bis 3,5.

Die Erfindung ist anhand der nachstehenden Beispiele erläutert. Es wurden folgende Komponenten verwendet :

(a) Nichtedelmetall-Legierungen (auch für Unterbauten geeignet)

|  | Ni | Cr | Fe | Al | Mo | Si | Be | Cu | Mn | Co |
|---|---|---|---|---|---|---|---|---|---|---|
| 1. | 69 | 17 | 0,5 | 4 | 5 | 1 | — | — | 3 | 0,5 |
| 2. | 81 | 12 | 0,2 | 3 | 1,9 | 0,2 | 1,5 | 0,1 | 0,1 | — |
| 3. | 0,3 | 31,6 | 0,6 | 0,01 | 4,4 | 1 | — | 0,01 | 0,78 | 61,3 |

Die vorstehend angegebenen Legierungen sind unter den Handelsbezeichnungen Wiron S (1), Gemini II (2) und Vitallium (3) im Handel erhältlich.

(b) Edelmetall-Legierungen

|  | Au | Pd | Pt | Ag | andere Metalle |
|---|---|---|---|---|---|
| 1. | 87,5 | 6 | 4,5 | — | 2 |
| 2. | 52,7 | 27 | — | 16 | 4,3 |

(c) Borax-Pulver
(d) Al-Pulver
(e) $SiO_2$-Pulver < 50 μm wahlweise
(f) $ZrO_2$-Pulver < 50 μm

Es werden verschiedene aufsinterbare Grundmassen hergestellt, wobei die Grundmassen A bis G unter Verwendung der in den vorstehend angegebenen Tabellen genannten Komponenten a1 bis a3, b1, b2 und d hergestellt wurden. Zur Herstellung der Grundmassen H bis M wurden statt der Komponenten b1 und b2 reines Gold bzw. andere Goldlegierungen verwendet. während statt des Al-Pulvers (Komponente d) bei den Grundmassen H bis L eine Al-Ag-Legierung verwendet wurde. Die verwendeten Komponenten sind in der nachstehenden Tabelle angegeben.

Komponenten

| Grund-masse | a1 | a2 | a3 | b1 | b2 | c | d | e | f |
|---|---|---|---|---|---|---|---|---|---|
| A | 35 | | | 42 | | 21 | 1,8 | 0,2 | — |
| B | | 35 | | | 35 | 25 | 5 | — | — |
| C | | | 35 | 35 | | 15 | 15 | — | — |
| D | 10 | | | | 40 | 20 | 10 | 8 | 12 |
| E | 35 | | | 35 | | 15 | 5 | 10 | — |
| F | | 33 | | | 22 | 20 | 10 | 15 | — |
| G | | | 32 | | 38 | 15 | 8 | — | 7 |

| | | | | Au | Ag | Cu | | Al | Ag | | |
|---|---|---|---|---|---|---|---|---|---|---|---|
| H | 28,69 | | | 39,15 | 2,0 | — | 10,65 | — | — | 10,97 | 8,57 |
| I | 35,47 | | | 18,14 | 0,96 | — | 15,83 | 2,44 | 0,43 | 20,37 | 6,36 |
| J | 38,74 | | | 27,15 | 5,09 | 1,7 | 17,25 | 2,66 | 0,47 | — | 6,94 |
| K | 46,13 | | | 19,95 | 1,05 | — | 13,05 | 2,66 | 0,47 | 16,69 | — |
| L | 43,92 | | | 24,03 | 3,4 | 1,13 | 22,18 | 4,54 | 0,8 | — | — |
| M | 39,0 | | | 38,0 | — | — | 20,0 | 3,0 | — | — | — |

Diese Grundmassen wurden jeweils mit einer Trägerflüssigkeit (Glycerin-Äthanol 1 : 1) zu einer cremigen Konsistenz gemischt. Mit einem Pinsel wurden die Grundmassen in einer möglichst gleichmäßigen Stärke von etwa 0,4 mm auf Metallplättchen mit der Legierungszusammensetzung (a1) aufgetragen (Abmessungen 15 × 7 × 0,5 mm). Dann wurde die Grundmasse gut vorgetrocknet und in einem Brennofen unter Vakuum bei 950 °C 2 Minuten gesintert. Die gebrannten Metallplättchen wurden abgekühlt und anschließend 10 Minuten in einem Ultraschallbad mit einer Säurelösung (10 % HCl, 10 % HF) in Wasser behandelt.

Nach der Entnahme aus dem Ultraschallbad und gründlichem Spülen unter Wasser wurde eine 1,5 mm starke Zahnporzellanschicht aufgebrannt. Die Metallplättchen mit dem Zahnporzellan wurden zu einem Ring mit einem Umfang von 15 mm geformt und anschließend wieder in ihre Originalform gebracht. Nach dem Geradestrecken wurde das Zahnporzellan auf dem Metallplättchen mit einem scharfen Luftstrahl angeblasen, um loses Porzellan zu entfernen. Die Metallplättchen wurden dann unter einem Mikroskop betrachtet. Bei Verwendung der erfindungsgemäßen Grundmassen A bis M wurde festgestellt, daß eine Zahnporzellanschicht auf dem Metall haften blieb.

Zum Vergleich wurde auf die Legierung (a1) in der gleichen Weise Zahnporzellan aufgebrannt, wobei zuvor keine erfindungsgemäße Grundmasse aufgesintert wurde. Wurde das so erhaltene Metallplättchen in der gleichen Weise gebogen und wieder geradegesteckt, so splitterte der größte Teil des Zahnporzellans ab bzw. es erfolgte von Anfang an keine Haftung.

## Ansprüche

1. Aufsinterbare Grundmasse zur Herstellung einer Zwischenschicht zwischen einer hochschmelzenden Dentalmetall-Legierung, insbesondere einer Nichtedelmetall-Legierung, und Zahnporzellan, gekennzeichnet durch die Komponenten :

(a) mindestens ein hochschmelzendes, unedles Dentalmetall oder eine hochschmelzende Dentalmetall-Legierung ;

(b) Gold bzw. eine Goldverbindung, die sich zu metallischem Gold zersetzt, oder eine Goldlegierung ;

(c) mindestens ein Flußmittel ; und

(d) Aluminium und/oder Silber oder eine Aluminium-Silber-Legierung bzw. eine Silberverbindung, die sich zu metallischem Silber zersetzt.

2. Grundmasse nach Anspruch 1, dadurch gekennzeichnet, daß sie zusätzlich

(e) Kieselsäure und/oder

(f) Zirkondioxid enthält.

3. Grundmasse nach Anspruch 1 oder 2, dadurch gekennzeichnet, daß mindestens die Komponenten (a), (b) und (d), sowie gegebenenfalls die Komponenten (e) und (f) in Form von Teilchen vorliegen.

4. Grundmasse nach einem der Ansprüche 1 bis 3, dadurch gekennzeichnet, daß die Teilchengröße der Komponenten zwischen etwa 10 und 200 μm, vorzugsweise zwischen etwa 20 und 60 μm liegt.

5. Grundmasse nach Anspruch 4, dadurch gekennzeichnet, daß die Komponenten folgende Teilchengrößen (in μm) haben :

(a) 20 bis 50
(b) 30 bis 200, vorzugsweise weniger als 150
(c) weniger als 150
(d) 20 bis 200, vorzugsweise weniger als 50
(e) 20 bis 150
(f) weniger als 50

6. Grundmasse nach einem der Ansprüche 1 bis 5, gekennzeichnet durch die Zusammensetzung (in Gew.-%) :

(a) 10 bis 70 ; (b) 10 bis 65 ; (c) 5 bis 50 ; (d) 0,6 bis 20 ; (e) 0 bis 30 ; (f) 0 bis 12.

7. Grundmasse nach einem der Ansprüche 1 bis 6, gekennzeichnet durch die Zusammensetzung (in Gew.-%) :

(a) 20 bis 50 ; (b) 35 bis 50 ; (c) 15 bis 30 ; (d) 1,5 bis 2 ; (e) 0 bis 20 ; (f) 0 bis 8.

8. Grundmasse nach einem der Ansprüche 1 bis 7, dadurch gekennzeichnet, daß die Komponente (a) Kobalt oder Chrom bzw. Gemische davon enthält.

9. Grundmasse nach Anspruch 8, dadurch gekennzeichnet, daß die Komponente (a) folgende Zusammensetzung (in Gew.-%) hat :

Cr 10 bis 20 ; Mo 1 bis 7 ; Al 1 bis 5 ; Si 0,5 bis 2 ; Mn 2 bis 5 ; Fe 0 bis 5 ; Co 0,1 bis 1 ; Ni-Rest.

10. Grundmasse nach Anspruch 9, dadurch gekennzeichnet, daß die Komponente (a) folgende Zusammensetzung (in Gew.-%) hat :

Cr 17 ; Mo 5 ; Al 4 ; Si 1 ; Mn 3 ; Fe 0,5 ; Co 0,5 ; Ni 69.

11. Grundmasse nach einem der Ansprüche 1 bis 10, dadurch gekennzeichnet, daß die Komponente (b) folgende Zusammensetzung (in Gew.-%) hat :

Au 50 bis 100 ; Ag 0 bis 25 ; Cu 0 bis 15 ; Pt 0 bis 10 ; Pd 0 bis 10.

12. Grundmasse nach Anspruch 11, dadurch gekennzeichnet, daß die Komponente (b) folgende Zusammensetzung (in Gew.-%) hat :

Au 76 ; Ag 15 ; Cu 8 ; Pt 0,4 ; Pd 0,6.

13. Grundmasse nach einem der Ansprüche 1 bis 12, dadurch gekennzeichnet, daß mindestens ein Teil der Komponenten (a) bis (f) in einer Trägerflüssigkeit (A) suspendiert ist.

14. Verfahren zur Herstellung einer Zwischenschicht zwischen einer hochschmelzenden Dentalmetall-Legierung, insbesondere einer Nichtedelmetall-Legierung, und Zahnporzellan, dadurch gekennzeichnet, daß man eine Grundmasse nach einem der Ansprüche 1 bis 13 mit einer Trägerflüssigkeit (A), in der die Komponente (c) mindestens teilweise löslich ist, vermischt, die Mischung auf die hochschmelzende Dentalmetall-Legierung aufbringt, trocknet und aufsintert und die aufgesinterte Grundmasse mit einer Flüssigkeit (B), in der die nichtmetallischen Komponenten der Grundmasse mindestens teilweise löslich sind, behandelt.

15. Verfahren nach Anspruch 14, dadurch gekennzeichnet, daß man als Trägerflüssigkeit (A) ein Gemisch aus Glycerin und Äthanol verwendet.

16. Verfahren nach Anspruch 15, dadurch gekennzeichnet, daß das Gewichtsverhältnis zwischen Glycerin und Äthanol in der Trägerflüssigkeit (A) etwa 1 : 1 beträgt.

17. Verfahren nach einem der Ansprüche 13 bis 16, dadurch gekennzeichnet, daß man als Flüssigkeit (B) eine verdünnte Mineralsäure oder ein Gemisch aus verdünnten Mineralsäuren verwendet.

18. Verfahren nach Anspruch 17, dadurch gekennzeichnet, daß die verdünnte Mineralsäure etwa 5-10 % HCl und/oder etwa 5-10 % HF in wäßriger Lösung enthält.

19. Verfahren nach einem der Ansprüche 14 bis 18, dadurch gekennzeichnet, daß man die Suspension der Grundmasse in einer Schichtstärke von etwa 0,2 bis 0,5 mm auf die hochschmelzende Dentalmetall-Legierung aufbringt und bei ansteigenden Temperaturen bis zu etwa 800 bis 1 200 °C, vorzugsweise im Vakuum, aufsintert.

## 0 055 383

20. Verfahren nach einem der Ansprüche 14 bis 19, dadurch gekennzeichnet, daß man als hochschmelzende Metallkomponente (a) eine Legierung verwendet, deren thermischer Ausdehnungskoeffizient dem des Zahnporzellans angepaßt ist.

21. Verfahren nach einem der Ansprüche 14 bis 20, dadurch gekennzeichnet, daß man als hochschmelzende Metallkomponente (a) eine Legierung mit Nickel, Kobalt oder Chrom bzw. Gemischen davon als Hauptbestandteile verwendet.

22. Verfahren nach Anspruch 21, dadurch gekennzeichnet, daß die hochschmelzende Metallkomponente (a) folgende Zusammensetzung (in Gew.-%) hat :

Ni und/oder Co 60 bis 85 ; Cr 11 bis 32 ; Fe 0,2 bis 0,6 ; Al 0 bis 5 ; Mo 1,5 bis 6 ; Si 0 bis 1,2 ; Be 0 bis 1,6 ; Cu 0 bis 0,2 ; Mg 0 bis 3,5.

**Claims**

1. A sinterable matrix for the production of an intermediate layer between a high-melting dental metal alloy, more particularly a non-precious metal alloy, and tooth porcelain, characterised by the following components :

(a) at least one high-melting non-precious dental metal or a high-melting dental metal alloy ;
(b) gold or a gold compound which decomposes to form metallic gold, or a gold alloy ;
(c) at least one flux ; and
(d) aluminium and/or silver or an aluminium-silver alloy or a silver compound which decomposes to form metallic silver.

2. A matrix according to claim 1, characterised in that it additionally contains :
(e) silicic acid and/or
(f) zirconium dioxide.

3. A matrix according to claim 1 or 2, characterised in that at least the components (a), (b) and (d) and if required the components (e) and (f), are in particle form.

4. A matrix according to any one of claims 1 to 3, characterised in that the particle size of the components is between approximately 10 and 200 μm, preferably between approximately 20 and 60 μm.

5. A matrix according to claim 4, characterised in that the components have the following particle sizes (in μm) :

(a) 20 to 50
(b) 30 to 200, preferably less than 150
(c) less than 150
(d) 20 to 200, preferably less than 50
(e) 20 to 150
(f) less than 50.

6. A matrix according to any one of claims 1 to 5, characterised by the following composition (in percent by weight) :
(a) 10 to 70 ; (b) 10 to 65 ; (c) 5 to 50 ; (d) 0.6 to 20 ; (e) 0 to 30 ; (f) 0 to 12.

7. A matrix according to any one of claims 1 to 6, characterised by the following composition (in percent by weight) :

(a) 20 to 50 ; (b) 35 to 50 ; (c) 15 to 30 ; (d) 1.5 to 2 ; (e) 0 to 20 ; (f) 0 to 8.

8. A matrix according to any one of claims 1 to 7, characterised in that the component (a) contains cobalt or chromium or mixtures thereof.

9. A matrix according to claim 8, characterised in that the component (a) has the following composition (in percent by weight) :

Cr 10 to 20 ; Mo 1 to 7 ; Al 1 to 5 ; Si 0,5 to 2 ; Mn 2 to 5 ; Fe 0 to 5 ; Co 0,1 to 1 ; Ni-remainder.

10. A matrix according to claim 9, characterised in that the component (a) has the following composition (in percent by weight) :

Cr 17 ; Mo 5 ; Al 4 ; Si 1 ; Mn 3 ; Fe 0,5 ; Co 0,5 ; Ni 69.

11. A matrix according to any one of claims 1 to 10, characterised in that the component (b) has the following composition (in percent by weight) :

8

Au 50 to 100 ; Ag 0 to 25 ; Cu 0 to 15 ; Pt 0 to 10 ; Pd 0 to 10.

12. A matrix according to claim 11, characterised in that the component (b) has the following composition (in percent by weight) :

Au 76 ; Ag 15 ; Cu 8 ; Pt 0.4 ; Pd 0.6.

13. A matrix according to any one of claims 1 to 12, characterised in that at least some of the components (a) to (f) are suspended in a liquid vehicle (A).

14. A process for the production of an intermediate layer between a high-melting dental metal alloy, more particularly a non-precious metal alloy, and tooth porcelain, characterised in that a matrix according to any one of claims 1 to 13 is mixed with a liquid vehicle (A) in which the component (c) is at least partially soluble, the mixture is applied to, dried and sintered on, the high-melting dental metal alloy and the sintered-on matrix is treated with a liquid (B) in which the non-metallic components of the matrix are at least partially soluble.

15. A process according to claim 14, characterised in that the liquid vehicle (A) is a mixture of glycerin and ethanol.

16. A process according to claim 15, characterised in that the weight ratio between glycerin and ethanol in the liquid vehicle (A) is approximately 1 : 1.

17. A process according to any one of claims 13 to 16, characterised in that liquid (B) is a dilute mineral acid or a mixture of dilute mineral acids.

18. A process according to claim 17, characterised in that the dilute mineral acid contains approximately 5-10 % HCl and/or about 5-10 % HF in aqueous solution.

19. A process according to any one of claims 14 to 18, characterised in that the matrix suspension is applied in a layer thickness of approximately 0.2 to 0.5 mm to the high-melting dental metal alloy and sintered thereon at rising temperatures of up to about 800 to 1,200 °C, preferably in vacuo.

20. A process according to any one of claims 14 to 19, characterised in that the high-melting dental metal component (a) is an alloy whose coefficient of thermal expansion is adapted to that of the tooth porcelain.

21. A process according to any one of claims 14 to 20, characterised in that the high-melting metal component (a) is an alloy containing nickel, cobalt or chromium or mixtures thereof as the main constituents.

22. A process according to claim 21, characterised in that the high-melting metal component (a) has the following composition (in percent by weight) :

Ni and/or Co 60 to 85 ; Cr 11 to 32 ; Fe 0.2 to 0.6 ; Al 0 to 5 ; Mo 1.5 to 6 ; Si 0 to 1.2 ; Be 0 to 1.6 ; Cu 0 to 0.2 ; Mg 0 to 3.5.

**Revendications**

1. Matière de base agglomérable par frittage, destinée à la préparation d'une couche intermédiaire entre un alliage métallique dentaire à point de fusion élevé, notamment un alliage de métaux non-précieux, et de la porcelaine dentaire, caractérisée par les composants suivants :

a) au moins un métal dentaire non-précieux ou alliage métallique dentaire à point de fusion élevé ;
b) de l'or ou un composé de l'or se décomposant en or métallique ou un alliage d'or ;
c) au moins un fondant ; et
d) de l'aluminium et/ou de l'argent, ou un alliage d'aluminium et d'argent ou un composé de l'argent se décomposant en argent métallique.

2. Matière de base selon la revendication 1, caractérisée en ce qu'elle renferme en outre :

e) de l'acide silicique et/ou
f) du dioxyde de zirconium.

3. Matière selon la revendication 1 ou 2, caractérisée en ce qu'au moins les composants a), b) et d), ainsi qu'éventuellement les composants e) et f), se présentent sous forme de particules.

4. Matière selon l'une des revendications 1 à 3, caractérisée en ce que la dimension granulométrique des composants est comprise entre environ 10 et 200 microns, de préférence entre environ 20 et 60 microns.

5. Matière selon la revendication 4, caractérisée en ce que les composants présentent les dimensions granulométriques suivantes (en microns) :

a) 20 à 50 ;
b) 30 à 200, de préférence moins de 150 ;

c) moins de 150 ;
d) 20 à 200, de préférence moins de 50 ;
e) 20 à 150 ;
f) moins de 50.

6. Matière selon l'une des revendications 1 à 5, caractérisée par la composition suivante (en pourcentage en poids) :

a) 10 à 70 ;
b) 10 à 65 ;
c) 5 à 50 ;
d) 0,6 à 20 ;
e) 0 à 30 ;
f) 0 à 12.

7. Matière selon l'une quelconque des revendications 1 à 6, caractérisée par la composition suivante (en % en poids) :

a) 20 à 50 ;
b) 35 à 50 ;
c) 15 à 30 ;
d) 1,5 à 2 ;
e) 0 à 20 ;
f) 0 à 8.

8. Matière selon l'une des revendications 1 à 7, caractérisée en ce que le composant a) renferme du cobalt ou du chrome, ou bien des mélanges de ceux-ci.

9. Matière selon la revendication 8, caractérisée en ce que le composant a) présente la composition suivante (en % en poids) :

Cr 10 à 20 ; Mo 1 à 7 ; Al 1 à 5 ; Si 0,5 à 2 ; Mn 2 à 5 ; Fe 0 à 5 ; Co 0,1 à 1 ; Ni, le complément.

10. Matière selon la revendication 9, caractérisée en ce que le composant a) présente la composition suivante (en % en poids) :

Cr 17 ; Mo 5 ; Al 4 ; Si 1 ; Mn 3 ; Fe 5 ; Co 0,5 ; Ni 69.

11. Matière selon l'une des revendications 1 à 10, caractérisée en ce que le composant b) présente la composition suivante (en % en poids) :

Au 50 à 100 ; Ag 0 à 25 ; Cu 0 à 15 ; Pt 0 à 10 ; Pd 0 à 10.

12. Matière selon la revendication 11, caractérisée en ce que le composant b) présente la composition suivante (en % en poids) :

Au 76 ; Ag 15 ; Cu 8 ; Pt 0,4 ; Pd 0,6.

13. Matière selon l'une des revendications 1 à 12, caractérisée en ce qu'une partie au moins des composants a) à f) est en suspension dans un liquide de support (A).

14. Procédé de préparation d'une couche intermédiaire entre un alliage de métal dentaire à point de fusion élevé, en particulier un alliage de métaux non-précieux, et de la porcelaine dentaire, caractérisé en ce qu'on mélange une matière de base selon l'une des revendications 1 à 13 avec un liquide de support (A) dans lequel le composant c) est au moins partiellement soluble, on applique le mélange à l'alliage métallique dentaire à point de fusion élevé, on le sèche et on l'agglomère par frittage, et l'on traite la matière de base agglomérée par frittage par un liquide (B) dans lequel les composants non-métalliques de la matière de base sont au moins partiellement solubles.

15. Procédé selon la revendication 14, caractérisé en ce qu'on utilise, comme liquide de support (A), un mélange de glycérine et d'éthanol.

16. Procédé selon la revendication 15, caractérisé en ce que le rapport pondéral entre la glycérine et l'éthanol dans le liquide de support (A) est d'eviron 1 : 1.

17. Procédé selon l'une des revendications 13 à 16, caractérisé en ce qu'on utilise, comme liquide (B), un acide minéral dilué ou un mélange d'acides minéraux dilués.

18. Procédé selon la revendication 17, caractérisé en ce que l'acide minéral dilué renferme environ 5 à 10 % de HCl et/ou environ 5 à 10 % de HF en solution aqueuse.

19. Procédé selon l'une des revendications 14 à 18, caractérisé en ce qu'on applique la suspension de la matière de base sur une épaisseur de couche d'environ 0,2 à 0,5 mm à l'alliage métallique dentaire à point de fusion élevé et on l'agglomère par frittage, de préférence sous vide, à des températures croissantes pouvant atteindre environ 800 à 1.200 °C.

20. Procédé selon l'une des revendications 14 à 19, caractérisé en ce qu'on utilise, comme composant métallique à point de fusion élevé a), un alliage dont le coefficient de dilatation thermique est adapté à celui de la procelaine dentaire.

21. Procédé selon l'une des revendications 14 à 20, caractérisé en ce qu'on utilise, comme composant métallique à point de fusion élevé a), un alliage avec le nickel, le cobalt ou le chrome, ou des mélanges de ceux-ci, comme constituants principaux.

22. Procédé selon la revendication 21, caractérisé en ce que le composant métallique à point de fusion élevé a) présente la composition suivante (en % en poids) :

Ni et/ou Co 65 à 85 ; Cr 11 à 32 ; Fe 0,2 à 0,6 ; Al 0 à 5 ; Mo 1,5 à 6 ; Si 0 à 1,2 ; Be 0 à 1,6 ; Cu 0 à 0,2 ; Mg 0 à 3,5.